# EUROPEAN PATENT APPLICATION

(11) **EP 4 410 269 A1**
(43) Date of publication of application: **07.08.2024**
(21) Application number: 22873974.4
(22) Date of filing: 05.08.2022
(51) Int. Cl.: A61K 8/29, A61Q 1/12, A61Q 17/04, A61Q 19/10

(54) **POWDER FOR COSMETIC COMPOSITION BLENDING, SAID POWDER BEING COMPOSED OF CALCIUM TITANIUM COMPOSITE OXIDE**

(30) Priority: 01.10.2021 JP 2021162842
(71) Applicant: Titan Kogyo Kabushiki Kaisha, Ube-shi Yamaguchi 755-8567 (JP)
(72) Inventor: MINO, Wataru, Ube-shi, Yamaguchi 755-8567 (JP); SHIMOMURA, Naotaka, Ube-shi, Yamaguchi 755-8567 (JP); NADA, Chihiro, Ube-shi, Yamaguchi 755-8567 (JP)
(74) Representative: Cabinet Beau de Loménie
(86) International application number: PCT/JP2022/030046
(87) International publication number: WO 2023/053720

(57) **Abstract**

As a material which is to be blended into a cosmetic composition that has UV-B blocking ability, while having high transparency without taking on a blue tinge, the present invention provides a powder which is to be blended into a cosmetic composition and is composed of a calcium titanium composite oxide, while having an average particle diameter of 25 nm to 110 nm. It is preferable that this powder is blended into a cosmetic composition in such an amount that the content of the calcium titanium composite oxide in the cosmetic composition is 300 g/kg or less.

## Description

### TECHNICAL FIELD

The present invention relates to a calcium-titanium composite oxide for use in blending into a cosmetic composition.

### BACKGROUND ART

Sunburn is a serious problem when applying makeup. Sunburn refers to symptoms such as pigmentation and inflammation in human skin caused by being exposed to ultraviolet rays, which significantly impairs the appearance. It is not too much to say that the effectiveness of sun protection measures is the key to the impression, especially when UV rays are strong, such as in summer. According to Japan Cosmetic Industry Association, among the light commonly referred to as ultraviolet rays, the light having a wavelength of 280 nm to 320 nm, which is called UV-B, significantly impairs the appearance by causing red inflammation on the skin, spots, and freckles.

To protect the skin from UV-B, an ultraviolet protection agent consisting of titanium oxide and zinc oxide has been conventionally used. Japanese Patent Laid-Open No. S62-228006 (PTL 1) discloses that a cosmetic with an ultraviolet protection effect is obtained by using zinc oxide having an average particle size of 70 to 300 µm in combination with fine particulate titanium oxide having an average particle size of 30 to 70 µm. However, zinc oxide and titanium oxide are white in color and have high hiding power, which may give an unnatural impression as if the applied area, especially the face, looks conspicuously white. This phenomenon is called "white cast" and is a serious problem for users. PTL 1 also mentions adverse effects of increased hiding power but does not sufficiently discuss the problem. An effective means of preventing the white cast is to reduce the particle size of the ultraviolet protection agent to improve the transparency. Japanese Patent Laid-Open No. 2001-262119 (PTL 2) describes in Example 2 that a compound in which Al is dissolved in ZnO having an average secondary particle size of 0.66 µm is obtained, the compound having both excellent transparency and ultraviolet absorption.

In the case of using a cosmetic with an ultraviolet protection agent having a small particle size, on the other hand, a cosmetic composition may show a faint blue tinge despite no blue colorant being blended therein. This phenomenon is still under debate but presumably occurs because powders preferentially scatter light with a shorter wavelength among natural light through a phenomenon so-called Rayleigh scattering. Rayleigh scattering is described in NPL 1, for example. If the area to which the cosmetic composition has been applied shows a faint blue tinge, it gives the impression to the surrounding people that the health condition of the user is not excellent, thereby seriously impairing the intended use of the cosmetic. In Japanese Patent Laid-Open No. H08-48614 (PTL 3), 0.1 to 20 parts by weight of yellow titanium oxide are blended into makeup cosmetics containing 0.5 to 20 parts by weight of rod-shaped powders to cancel out the blue tinge from the rod-shaped powders. In this method, however, it is necessary to blend the rod-shaped powders with yellow titanium oxide, thereby increasing the types of powders to be added, so that a phenomenon is likely to occur in which the color of the cosmetic composition becomes uneven due to poor dispersion of the powders.

In addition, the use of titanium (IV) oxide, including rutile-type titanium oxide, and zinc oxide have been increasingly reduced or replaced by alternative materials, mainly in Europe, because of the undeniable health hazard potential of titanium (IV) oxide and the high impact of zinc oxide on aquatic life. Japanese Patent Laid-Open No. 2018-514509 (PTL 4) describes a UV protection composition containing barium titanate, and suggests from Fig. 1A that barium titanate can be substituted for titanium oxide TiO₂ as an ultraviolet absorber.

PTL 4 also describes that the sunscreen composition is preferably transparent in general and that coloring compositions obtained in some embodiments are substantially transparent but sometimes faintly colored. No mention is made of the specific color of the composition or of how to eliminate the blue tinge. Moreover, barium titanate is designated as a deleterious substance in Japan and is therefore not suitable for blending into cosmetic compositions.

### CITATION LIST

### PATENT LITERATURE

PTL 1: JP S62-228006 A
PTL 2: JP 2001-262119 A
PTL 3: JP H08-48614 A
PTL 4: JP 2018-514509 A

### NON PATENT LITERATURE

NPL 1: NAKAHARA, Masayoshi, Iro no Kagaku (Color Science), 7th edition, BAIFLTKAN CO., LTD., 1989, p.14-16

### SUMMARY OF INVENTION

### TECHNICAL PROBLEM

There is a need for a material other than barium titanate that can replace titanium oxide and zinc oxide without taking on a blue tinge.

An object of the present invention is to provide a material that has the ability to block UV-B and, when blended into a cosmetic composition, high transparency and hardly leaves a white cast without taking on a blue tinge.

### SOLUTION TO PROBLEM

As a result of intensive studies on the above-mentioned material, the present inventors have found that a powder composed of a calcium-titanium composite oxide having an average particle size of 25 nm or more and 110 nm or less has the ability to block UV-B and leaves no white cast without taking on a blue tinge.

The present invention includes, but is not limited to, the following.
[1] A powder to be blended into a cosmetic composition, the powder containing calcium-titanium composite oxide particles having an average particle size of 25 nm or more and 110 nm or less.
[2] The powder according to [1], in which circularity of the particles is 0.80 or more.
[3] The powder according to [1] or [2], including a coating layer of an inorganic and/or organic substance on at least a part of a surface of the particles.
[4] A dispersion containing the powder according to any one of [1] to [3] and a disperse medium.
[5] A cosmetic composition containing the powder according to any one of [1] to [3] or the dispersion according to [4].
[6] A cosmetic composition containing the powder according to any one of [1] to [3] or the dispersion according to [4] in an amount such that a content of calcium-titanium composite oxide is 300 g/kg or less.

### ADVANTAGEOUS EFFECTS OF INVENTION

The cosmetic composition with the powder obtained in the present invention has excellent ability to block UV-B and can therefore be suitably used as a cosmetic composition that blocks UV-B.

Since the cosmetic composition with the powder obtained in the present invention has high transparency, it does not leave a white cast when applied to the skin, thereby giving the user a natural impression.

Furthermore, the cosmetic composition with the powder obtained in the present invention does not show a blue tinge. The blue tinge generally refers to a JIS common name of blue or a tinge close to it. Although there are large individual differences due to the user's physical characteristics and the observer's senses, in general, the presence of a part exhibiting these colors on the user's face gives the impression that the user's health is not excellent, thereby significantly impairing the intended use as a cosmetic. Since the cosmetic composition of the present invention does not show a blue tinge, it can give the user a healthy impression.

### DESCRIPTION OF EMBODIMENTS

The "powder composed of calcium-titanium composite oxide" obtained in the present invention means that most of particles constituting the powder are calcium-titanium composite oxide particles or that the proportion of calcium-titanium composite oxide is large in the composition of individual particles. The powder of the present invention is most preferably composed of pure calcium-titanium composite oxide containing no impurities at all in all of the particles but may contain unreacted products during synthesis reaction of the calcium-titanium composite oxide, unavoidable impurities derived from raw materials, and inorganic and/or organic substances derived from a coating layer, in addition to the calcium-titanium composite oxide. Specifically, if the whole powder is composed of 600 g/kg or more, preferably 650 g/kg or more, and more preferably 700 g/kg or more of calcium-titanium composite oxide, the powder may be regarded as a powder composed of a calcium-titanium composite oxide realizing the effects of the present invention.

The powder blended into the cosmetic composition of the present invention has an average particle size of particles constituting the powder of 110 nm or less, more preferably 100 nm or less, even more preferably 95 nm or less, and still more preferably 93 nm or less. When the average particle size is 110 nm or less, the powder does not leave a white cast without taking on a blue tinge. The average particle size is 25 nm or more, more preferably 30 nm or more, still more preferably 35 nm or more. When the average particle size is 25 nm or more, the probability of agglomeration occurring, which is a problem when the powder is blended and used in a cosmetic composition, is sufficiently small. The average particle size is evaluated by the method described below. Note that the average particle size in the present invention refers to a value obtained by rounding off the first decimal place.

The powder of the present invention has an effect of leaving no white cast without taking on a blue tinge when being blended into a cosmetic composition. Although the mechanism of such effect is not clear, the present inventors speculate that the small average particle size of the particles constituting the powder of the present invention and the moderate refractive index of the calcium-titanium composite oxide create an optical synergistic effect to suppress both the white cast and the blue tinge when used as a cosmetic.

The powder to be blended into the cosmetic composition of the present invention is not particularly limited in the particle size distribution of the constituent particles. However, the distribution of the particle sizes of the individual particles constituting the powder is preferably narrow in the present invention. Most preferably, the histogram of the number of particles plotted with respect to the particle size has a single peak. As a rough standard, the standard deviation of the particle size of about 300 particles measured by the method described later is preferably less than 15.0 nm.

In the powder to be blended into the cosmetic composition of the present invention, the particles constituting the powder each preferably have a circularity of 0.80 or more, more preferably 0.81 or more. When the circularity is 0.80 or more, a smooth feel can be achieved. There is no particular upper limit to the circularity of the particles. The theoretical maximum is 1.00. The circularity is evaluated by the method described below.

The powder to be blended into the cosmetic composition of the present invention is not particularly limited in variation in circularity and shape. However, it is desirable that the proportion of particles having the same circularity and/or the same shape is large. Specifically, the standard deviation of the circularity of about 200 particles measured by the method described later is preferably less than 0.07.

It is desirable that the powder to be blended into the cosmetic composition of the present invention includes a coating layer on at least a part of the surface of the particles constituting the powder to impart, for example, hydrophobicity and optical properties. The coating layer may include a coating layer composed of organic substance or a coating layer composed of inorganic substance. Two or more coating layers may be present, or both organic and inorganic layers may be present. The coating layer is described in detail below.

The powder obtained in the present invention usually contains no titanium oxide or has a small content thereof. The cosmetic composition using the powder obtained in the present invention can be used as a cosmetic containing no titanium oxide or having a small content of titanium oxide. Specifically, the titanium oxide content of the powder is preferably 200 g/kg or less, more preferably 100 g/kg or less. The content can be evaluated by a method including comparing the ratio of the peak intensity derived from titanium oxide and the peak intensity derived from the calcium-titanium composite oxide obtained by X-ray diffraction measurement, with a calibration curve. The calibration curve is created by measuring and plotting at least three points of a mixture of the calcium-titanium composite oxide and titanium oxide whose mixing ratio is known.

The powder to be blended into the cosmetic composition of the present invention is desirably dispersed in a disperse medium and used as a dispersion. The dispersion can be used in cosmetic compositions such as sunscreens and sun creams.

The powder obtained in the present invention is desirably blended into a cosmetic composition. Although the dosage form of the cosmetic composition is not particularly limited, it is typically desirable that the cosmetic composition is skin cosmetics such as sunscreens.

When the powder or dispersion obtained in the present invention is blended into a cosmetic composition, the blending amount thereof should be such that the amount of calcium-titanium composite oxide in the cosmetic composition is 300 g/kg or less, preferably 270 g/kg or less. If the above blending amount is 300 g/kg or less, the cosmetic composition gives a smooth feel and can also maintain higher transparency. The lower limit of the blending amount is not particularly limited. In general, when the blending amount is 45 g/kg or more, a sufficient ultraviolet protection effect can be imparted to the cosmetic without separately adding an ultraviolet protection agent. The blending amount is more preferably 50 g/kg or more. When used in combination with other ultraviolet protection agents, when blended into cosmetic compositions for which ultraviolet protection effect is not desired, or the like, the amount may be smaller. In such a case, the lower limit of the blending amount is 10 g/kg, more preferably 20 g/kg, as a rough standard.

### (Production Method)

The method for producing the powder to be blended into the cosmetic composition of the present invention is not particularly limited. Typically, the powder can be obtained by mixing a titanium source represented by metatitanic acid and a calcium source represented by calcium hydroxide, heating the mixture, and removing excess calcium.

In the production of the powder composed of calcium-titanium composite oxide particles to be blended into the cosmetic composition of the present invention, one or more sugars selected from monosaccharides such as glucose and fructose and disaccharides such as maltose may be added to the raw material. When such sugar is added, the amount of the sugar added is preferably 0.500 mol or less in total with respect to titanium. When the amount of the sugar added is 0.500 mol or less, sugar and titanium oxide hardly remain. Although the particles generally tend to be small when the amount of sugar added is large, the particles are preferably not too small when blended into the cosmetic composition, and from this point of view, the concentration of added sugar is preferably 0.500 mol or less in total with respect to titanium. The lower limit is not particularly limited but is preferably 0.030 mol or more, more preferably 0.031 mol or more with respect to titanium. If calcium is not dissolved in water, the addition of sugar is not necessary.

The powder to be blended into the cosmetic composition of the present invention can be produced by a reaction under high temperature and high pressure using an autoclave.

The powder to be blended into the cosmetic composition of the present invention can also be produced by mixing a titanium source and a calcium source and firing at a high temperature.

The powder to be blended into the cosmetic composition of the present invention may be synthesized by a method other than the method above. For example, the powder to be blended into the cosmetic composition of the present invention may be produced with reference to a method of adding a metal compound and an alcohol to a titanium peroxo complex dispersion and subjecting the mixture to heat treatment, which is described in Japanese Patent Laid-Open No. 2020-075846. The powder to be blended into the cosmetic composition of the present invention may also be produced with reference to a method of causing a hydrothermal reaction of calcium ions and titanium ions with other ions in water in a supercritical or subcritical state, which is described in Japanese Patent Laid-Open No. 2008-303131.

### (Surface Coating Treatment)

The powder to be blended into the cosmetic composition of the present invention may be provided with a coating layer of an inorganic substance such as a hydrous oxide or oxide of a metal such as aluminum, silicon, zinc, titanium, zirconium, iron, cerium, and tin on at least a part of the surface of the particles constituting the powder, for example, to impart dispersion stability and optical properties in a dispersion medium. A metal salt other than the above may be used for the inorganic coating. In addition, an organic coating layer may be provided on at least a part of the surface of the particles to perform surface modification represented by hydrophobic treatment. Examples of organic coatings include treating with a silicone compound such as dimethylpolysiloxane, hydrogen dimethicone, and polysiloxane; a coupling agent such as a silane coupling agent, an aluminum coupling agent, a titanium coupling agent, and a zirconium coupling agent; a fluorine compound such as a perfluoroalkyl phosphate compound; a hydrocarbon; lecithin; an amino acid; polyethylene; a wax; and a metal soap. A plurality of these treatments may be carried out in combination, and the order of the treatments is not particularly limited. The method of the surface treatment is not particularly limited, and a conventional method may be used. For example, the coating layer can be formed using a method of mixing the material of the coating layer with powder, followed by heat treatment, or the like.

### (Dispersion)

The powder to be blended into the cosmetic composition of the present invention is desirably used in the form of a dispersion in which the powder is dispersed in a disperse medium. The type of the disperse medium may be an aqueous dispersion medium or an oily dispersion medium without particular limitation. Examples of the aqueous dispersion medium include water, ethanol, isobutyl alcohol, propanediol, phenoxyethanol, and polyvinyl alcohol, and examples of the oily dispersion medium include aromatic oil, olive oil, glycerin, squalane, candelilla wax, 1,3-butylene glycol, pentylene glycol, toluene, vaseline, castor oil, lanolin, beeswax, isododecane, paraffin, and silicone. The method of dispersing is not particularly limited. Specifically, devices such as a homogenizer, a homomixer, and a bead mill can be used according to the amount and viscosity of the disperse medium. Depending on the type and amount of disperse medium, it is desirable to perform a surface coating treatment before dispersion.

### (Cosmetic Composition)

The powder obtained in the present invention can be used by being blended into a cosmetic composition and in particular, is preferably blended into skin cosmetics and used as a sunscreen by taking advantage of its ability to block UV-B. Besides, the powder can be used in a wide range of applications such as foundation, a skin care product, makeup base, and suntan lotion.

The cosmetic composition with the powder obtained in the present invention has excellent ability to block UV-B and can therefore be suitably used as a cosmetic composition that blocks UV-B. The smaller the transmittance integral in the UV-B region, the greater the ability to block UV-B, in particular, if the transmittance integral in the UV-B region is less than 800%•nm, it can be determined that the cosmetic composition has a sufficient ability to block UV-B. In the present invention, the UV-B region is defined in the range of 280 nm or more and 320 nm or less.

Since the cosmetic composition with the powder obtained in the present invention has high transparency, it does not leave a white cast when applied to the skin, thereby giving the user a natural impression. The greater the transmittance integral of light in a human-recognizable region (hereinafter referred to as "visible region"), the higher the transparency. In particular, if the transmittance integral in the visible region is more than 35000%•nm, it can be determined that the transparency is sufficiently high. In the present invention, the visible region is defined in the range of 380 nm or more and 800 nm or less.

Furthermore, the cosmetic composition with the powder obtained in the present invention does not show a blue tinge. The greater the transmittance integral of light in a region exhibiting purple and blue (hereinafter referred to as "blue region"), the lower the intensity of bluish scattered light, resulting in a less blue tinge. In particular, if the transmittance integral in the blue region is more than 9000%•nm, it can be determined that the cosmetic composition does not leave a blue tinge. Furthermore, it is more preferable that the value obtained by dividing the transmittance integral in the blue region by the transmittance integral in the visible region is more than 0.250. In the present invention, the blue region is defined in the range of 380 nm or more and 500 nm or less.

The blue tinge generally refers to a JIS common name of blue or a tinge close to it. Although there are big differences due to the user's physical characteristics and the observer's senses, in general, the presence of a part exhibiting these colors on the user's face gives the impression that the user's health is not excellent, thereby significantly impairing the intended use as a cosmetic. Since the cosmetic composition of the present invention does not show a blue tinge, it can give the user a healthy impression.

The cosmetic composition using the powder obtained in the present invention can be adjusted in color by addition of other powder (pigment) within a quantitative and qualitative range that does not impair the effects of the present invention. Examples of the inorganic pigment that can be usually used in combination include titanium oxide, zinc oxide, red iron oxide, yellow iron oxide, black iron oxide, ultramarine blue pigment, iron blue pigment, cerium oxide, talc, white mica, synthetic mica, brown mica, black mica, synthesized fluorinated brown mica, mica titanium, micaceous iron oxide, sericite, zeolite, kaolin, bentonite, clay, silicic acid, silicic anhydride, magnesium silicate, magnesium aluminum silicate, calcium silicate, barium sulfate, magnesium sulfate, calcium sulfate, calcium carbonate, magnesium carbonate, boron nitride, bismuth oxychloride, alumina, zirconium oxide, magnesium oxide, chromium oxide, calamine, hydroxyapatite, and a complex thereof. Examples of the organic pigment that can also be used in combination include a silicone powder, an elastic silicone powder, a polyurethane powder, a cellulose powder, a nylon powder, a urethane powder, a silk powder, a PMMA powder, starch, a polyethylene powder, a polystyrene powder, carbon black, tar dye, a natural dye, a metal soap such as zinc stearate, and a complex thereof.

To impart the ability to block UV-A or UV-C or to further enhance the ability to block UV-B, an ultraviolet protection agent may be separately added to the cosmetic composition using the powder obtained in the present invention within a quantitative and qualitative range that does not impair the effects of the present invention. The ultraviolet protection agent to be added may be either an organic ultraviolet protection agent or an inorganic ultraviolet protection agent. Note that the cosmetic composition using the powder obtained in the present invention has the ability to block UV-B required for general sunscreens and sun creams without separately adding an ultraviolet protection agent. Therefore, the cosmetic composition of the present invention may not contain an ultraviolet protection agent other than the powder of the present invention.

The cosmetic composition using the powder obtained in the present invention can also be blended with ingredients other than the aforementioned ingredients within a quantitative and qualitative range that does not impair the effects of the present invention, according to the intended use. For example, an oily ingredient, a dye, a pH adjusting agent, a moisturizer, a thickener, a surfactant, a dispersing agent, a stabilizer, a coloring agent, an antiseptic, an antioxidizing agent, a sequestering agent, an astringent, an anti-inflammatory agent, and a fragrance can also be appropriately blended within the ranges that achieve the object of the present invention. In particular, it is desirable to blend a plate-like compound represented by mica so as to improve slipperiness.

Examples of the dosage forms of the cosmetic with the powder obtained in the present invention are given below.

The cosmetic composition using the powder obtained in the present invention can be used as a liquid cosmetic. The dosage form may be any form including aqueous dispersions, oily dispersions, and lotions. Examples thereof include a sunscreen, a suntan lotion, a makeup base, a skin care cosmetic, and a hair care cosmetic.

The cosmetic composition using the powder obtained in the present invention can be formulated into a liquid cosmetic and then used by spraying. The form of the cosmetic when sprayed can be any form, such as foam and mist. Examples thereof include a spray-type sunscreen, a makeup base, a skin care cosmetic, and a hair care cosmetic.

The cosmetic composition using the powder obtained in the present invention can also be used as a gel cosmetic. The dosage form may be any form including creams, emulsions, oily liquids, and pastes. Examples thereof include a sunscreen, a makeup cosmetic such as a makeup base and a lipstick, and a hair care cosmetic. The cosmetic composition can also be used as body cleansing cosmetic compositions, such as shampoos, rinses, body shampoos, cleansing foams, peel-off masks, and toothpastes.

The cosmetic composition using the powder obtained in the present invention can also be used as a solid cosmetic. The dosage form may be any form including powders, powder solids, pastes, and oily solids. Examples thereof include a sunscreen, a makeup cosmetic such as a makeup base, a foundation, a loose powder, a concealer, a face powder, a body powder, a perfumed powder, and a baby powder, a skin care cosmetic, and a hair care cosmetic.

Regardless of the dosage form used, the series of characteristics of the powder obtained in the present invention, such as UV-B blocking, high transparency, and no blue tinge, are useful in many cosmetic dosage forms.

The cosmetic composition with the powder obtained in the present invention can be suitably used regardless of the gender of the user. In addition, the calcium-titanium composite oxide, which is the main component of the powder obtained in the present invention, has not been reported to cause any health hazard, and thus can be suitably used regardless of the age of the user.

The cosmetic composition using the powder obtained in the present invention can be suitably used for animals except for human beings. Typical applications include sun cream for domestic dogs. In such applications, there are cases where animals lick and ingest the powder. Therefore, the powder composed of the calcium-titanium composite oxide, which has not been reported to have health hazard, makes it useful when prescribing to animals other than human beings.

The powder obtained in the present invention can be used in applications other than cosmetics by taking advantage of its ability to block UV-B and high transparency. Typical applications include addition to plastic sun visors, which have become popular in recent years, to help protect against UV radiation, and use in house paints to mitigate UV-induced deterioration of exterior walls. In recent years, users who avoid products containing titanium oxide in various fields have increased due to growing health awareness, and products using the powder obtained in the present invention can satisfy the demands of such users.

Prior to describing Examples, test methods used in the present invention are described.

### [Particle Size and Average Particle Size]

Measurements were taken using a transmission electron microscope JEM-1400 plus manufactured by JEOL LIMITED. The observation magnification was set at 50,000 (10,000 (observation magnification of transmission electron microscopy) × 5 (printing magnification)). The diameters of about 300 particles in the observation field of view were evaluated using image analysis software ImageJ to determine the particle size. The average of particle sizes of about 300 particles was defined as the average particle size in the present invention.

### [Circularity]

The circularity was evaluated by projecting a particle into two dimensions and calculating (length of circumference of a circle having an area equal to the area of the particle)/(length of circumference of the particle). The average circularity of about 200 particles constituting the powder was used as the circularity in the present invention.

### [Specific Surface Area]

The specific surface area was measured by one point method for BET using Gemini VII 2390 manufactured by MICROMERITICS INSTRUMENT CORPORATION.

### [Transmittance Integral]

First, a dispersion containing the powder of the present invention was prepared by the following procedure.

In an automatic mortar ALG-200WD manufactured by NITTO KAGAKU Co., Ltd., 40.0 g of powder (without surface coating treatment) and 4.8 g of methylhydrogenpolysiloxane KF-9901 manufactured by Shin-Etsu Chemical Co., Ltd. were added, mixed at a pestle rotation speed of 100 rpm for 60 minutes, and thermally treated at 110°C for 3 hours to obtain a surface-treated powder.

Into the EasyNano RMB model manufactured by Aimex Co., Ltd., 20.0 g of surface-treated powder, 5.0 g of polyglyceryl-3 polydimethylsiloxyethyl dimethicone (KF-6106, manufactured by Shin-Etsu Chemical Co., Ltd.), 25.0 g of decamethylcyclopentasiloxane (KF-995, manufactured by Shin-Etsu Chemical Co., Ltd.), and 200.0 g of φ0.5 mm beads (zirconia ball YTZ(R)-0.5, manufactured by NIKKATO CORPORATION) were put and dispersed at a disk peripheral speed of 12 m/s for 120 minutes, and the beads were separated by filtration to obtain a dispersion.

A sample for spectroscopic evaluation was prepared based on the obtained dispersion. 3.75 g of the dispersion and 5.25 g of decamethylcyclopentasiloxane (KF-995, manufactured by Shin-Etsu Chemical Co., Ltd.) were stirred for 2 minutes at 5000 rpm using Labolution(R), manufactured by PRIMIX Corporation, equipped with a 25 mm disper serrated turbine. Thereafter, 6.00 g of pure water was slowly added and stirred at 5000 rpm for 5 minutes to prepare a W/O emulsion-type sunscreen as a simple cosmetic. The obtained sunscreen was applied onto a 60 mm × 60 mm × 3 mm quartz plate using a No. 3 bar coater and dried with a household hair dryer under hot air of 1200 W to prepare a sample for spectroscopic evaluation.

The resulting sample for spectroscopic evaluation was analyzed using a spectrophotometer V-670 manufactured by JASCO Corporation, with a data acquisition interval of 2 nm, a UV/Vis bandwidth of 2.0 nm, a scanning rate of 1000 nm/min, and a light source of D2/WI in the double beam system to measure the transmittance at wavelengths from 250 nm to 800 nm. From the measurement results, the transmittance in the region having a wavelength of 380 nm or more and 800 nm or less was integrated, and the resulting integral was used as the transmittance integral in the visible region. It can be considered that the larger the transmittance integral in the visible region is, the higher the transparency is. Each transmittance integral was determined in the same manner for the range of 380 nm or more and 500 nm or less and the range of 280 nm or more and 320 nm or less and defined as the transmittance integral in the blue region and the transmittance integral in the UV-B region, respectively.

By using the cosmetic composition as the above sunscreen, it is also possible to determine various transmittance integrals of the cosmetic composition itself.

### EXAMPLES

The present invention will be described in detail with reference to Examples below, which are provided for illustrative purposes only and are not intended to limit the scope of the invention.

In stirring operations described in Examples and Comparative Examples, the rotation speed for stirring was adjusted appropriately so that the entire liquid was uniformly mixed and that droplets were not scattered around in consideration of properties related to the behavior of the liquid during stirring, such as the amount of liquid, the viscosity of the liquid, and the shape of the container. For products such as sodium hydroxide, the company name of the manufacturer and distributor thereof will be omitted, in the case where the same effect can be achieved by using a product from any company as long as it is a general commercially available product.

### [Example 1]

Metatitanic acid obtained by a sulfuric acid method was subjected to a deironizing and bleaching treatment, and a sodium hydroxide aqueous solution was then added to adjust the pH to 9.0 to carry out desulfurization, followed by neutralization with hydrochloric acid to adjust the pH to 5.8. The resultant was filtered and washed with water to obtain a washed cake of metatitanic acid with a SO₃ content of 9.3 g/kg. After water was added to the washed cake to make a slurry with a Ti content of 2.13 mol/L, hydrochloric acid was added to adjust the pH to 1.4 to carry out a deflocculation treatment. 0.764 mol of metatitanic acid in terms of TiO₂, which is a deflocculated product, was collected and put into a reaction vessel. Calcium hydroxide was added thereto at a Ca/Ti molar ratio of 1.15, followed by addition of 30.05 g of glucose. Then, 0.90 mol of sodium hydroxide was added, followed by addition of water to make a total volume of 0.6 L, and the mixed solution was stirred for 30 minutes.

The mixed solution was heated to 150°C using an autoclave under further stirring and mixing, held with stirring for 10.0 hours, and then cooled to 50°C. Hydrochloric acid was added until the pH reached 5.5, and stirring was continued for another 1 hour. The resulting precipitate was washed by decantation, separated by filtration, and dried in the atmosphere at 120°C for 10 hours to obtain a powder composed of calcium-titanium composite oxide particles. The particles constituting the obtained powder had an average particle size of 31 nm, a circularity of 0.820, and a specific surface area of 116.4 m²/g, which were used to prepare a sample for spectroscopic evaluation, and the transmittance integral was evaluated. The results were as follows: the transmittance integral was 35913%•nm in the visible region, 9448%•nm in the blue region, and 622%•nm in the UV-B region.

### [Example 2]

A powder composed of calcium-titanium composite oxide particles was obtained in the same manner as in Example 1, except that the amount of metatitanic acid collected was changed to 0.675 mol in terms of TiO₂, the amount of glucose added was changed to 8.02 g, the amount of sodium hydroxide added after the addition of glucose was changed to 1.08 mol, and the holding time at 150°C was changed to 6.0 hours. The particles constituting the obtained powder had an average particle size of 62 nm, a circularity of 0.824, and a specific surface area of 74.2 m²/g, which were used to prepare a sample for spectroscopic evaluation, and the transmittance integral was evaluated. The results were as follows: the transmittance integral was 35682%•nm in the visible region, 9422%•nm in the blue region, and 601%•nm in the UV-B region.

### [Example 3]

A powder composed of calcium-titanium composite oxide particles was obtained in the same manner as in Example 1, except that the amount of metatitanic acid collected was changed to 0.675 mol in terms of TiO₂, the amount of glucose added was changed to 4.01 g, and the amount of sodium hydroxide added after the addition of glucose was changed to 1.08 mol. The particles constituting the obtained powder had an average particle size of 93 nm, a circularity of 0.814, and a specific surface area of 60.3 m²/g, which were used to prepare a sample for spectroscopic evaluation, and the transmittance integral was evaluated. The results were as follows: the transmittance integral was 35340%•nm in the visible region, 9113%•nm in the blue region, and 251%•nm in the UV-B region.

### [Example 4]

Metatitanic acid was fired in air at 500°C for 40 minutes. 0.021 mol of the resulting fired product of metatitanic acid in terms of TiO₂ was collected, and the resulting fired product of the metatitanic acid and 6.32 g of calcium carbonate was pulverized and mixed for 4 hours with an Ishikawa stirring and crushing machine AGA model manufactured by Ishikawa Kojo Co., Ltd. The pulverized and mixed raw materials were fired at 850°C for 40 minutes.

The fired product was washed in a solution prepared by adding hydrochloric acid to pure water, further washed with pure water, and then dried at 120°C in the atmosphere to obtain a powder composed of calcium-titanium composite oxide particles. The particles constituting the obtained powder had an average particle size of 37 nm, a circularity of 0.870, and a specific surface area of 42.9 m²/g, which were used to prepare a sample for spectroscopic evaluation, and the transmittance integral was evaluated. The results were as follows: the transmittance integral was 36220%•nm in the visible region, 9579%•nm in the blue region, and 374%•nm in the UV-B region.

### [Comparative Example 1]

A powder composed of calcium-titanium composite oxide particles was obtained in the same manner as in Example 1, except that the amount of metatitanic acid collected was changed to 0.675 mol in terms of TiO₂, the amount of glucose added was changed to 3.51 g, and the amount of sodium hydroxide added after the addition of glucose was changed to 1.08 mol. The particles constituting the obtained powder had an average particle size of 131 nm, a circularity of 0.837, and a specific surface area of 44.0 m²/g, which were used to prepare a sample for spectroscopic evaluation, and the transmittance integral was evaluated. The results were as follows: the transmittance integral was 33006%•nm in the visible region, 7942%•nm in the blue region, and 217%•nm in the UV-B region.

### [Comparative Example 2]

A powder composed of calcium-titanium composite oxide particles was obtained in the same manner as in Example 1, except that the amount of metatitanic acid collected was changed to 0.675 mol in terms of TiO₂, the amount of glucose added was changed to 2.76 g, the amount of sodium hydroxide added after the addition of glucose was changed to 1.08 mol, and the operation of heating was performed in the atmosphere at 95°C for a holding time of 18.0 hours. The particles constituting the obtained powder had an average particle size of 212 nm, a circularity of 0.826, and a specific surface area of 34.9 m²/g, which were used to prepare a sample for spectroscopic evaluation, and the transmittance integral was evaluated. The results were as follows: the transmittance integral was 32618%•nm in the visible region, 7671%•nm in the blue region, and 293%•nm in the UV-B region.

### [Comparative Example 3]

A powder composed of calcium-titanium composite oxide particles was obtained in the same manner as in Example 1, except that the amount of metatitanic acid collected was changed to 0.675 mol in terms of TiO₂, the amount of sodium hydroxide added in the subsequent step was changed to 1.08 mol without addition of glucose, and the operation of heating was performed in the atmosphere at 95°C for a holding time of 18.0 hours. The particles constituting the obtained powder had an average particle size of 311 nm, a circularity of 0.697, and a specific surface area of 16.8 m²/g, which were used to prepare a sample for spectroscopic evaluation, and the transmittance integral was evaluated. The results were as follows: the transmittance integral was 24013%•nm in the visible region, 4205%•nm in the blue region, and 493%•nm in the UV-B region.

### [Comparative Example 4]

A powder of cosmetic ultrafine titanium oxide ST-450EC manufactured by Titan Kogyo, Ltd. was used. This powder had a particle size of 38 nm, a circularity of 0.470, and a specific surface area of 51.3 m²/g, which was used to prepare a sample for spectroscopic evaluation, and the transmittance integral was evaluated. The results were as follows: the transmittance integral was 35191%•nm in the visible region, 8723%•nm in the blue region, and 3%•nm in the UV-B region.

### [Reference Example 1]

A commercially available W/O emulsion-type sunscreen was applied onto a quartz plate and dried to prepare a sample for spectroscopic evaluation, and the transmittance integral was evaluated. The results were as follows: the transmittance integral was 32396%•nm in the visible region, 8243%•nm in the blue region, and 953%•nm in the UV-B region.

**[Table 1]**

| | TiO₂ | Glucose/Ti | Ca/Ti | NaOH | Volume of reaction system | Heating temperature | Holding time |
|---|---|---|---|---|---|---|---|
| | mol | mol/mοl | mol/mol | mol | L | °C | h |
| Example 1 | 0.764 | 0.218 | 1.15 | 0.90 | 0.6 | 150 | 10.0 |
| Example 2 | 0.675 | 0.066 | 1.15 | 1.0 8 | 0.6 | 150 | 6.0 |
| Example 3 | 0.675 | 0.033 | 1.15 | 1.0 8 | 0.6 | 150 | 10.0 |
| Example 4 | 0.021 | 0.000 | 3.00 | 0.00 | - | 850 | 0.7 |
| Comparative Example 1 | 0.675 | 0.029 | 1.15 | 1.08 | 0.6 | 150 | 10.0 |
| Comparative Example 2 | 0.675 | 0.023 | 1.15 | 1.08 | 0.6 | 95 | 18.0 |
| Comparative Example 3 | 0.675 | 0.000 | 1.15 | 1.08 | 0.6 | 95 | 18.0 |
| Comparative Example 4 | - | - | - | - | - | - | - |

**[Table 2]**

| | Average particle size | Particle circularity | Particle specific surface area | Transmittance integral at each wavelength (performance characteristics) | | | |
|---|---|---|---|---|---|---|---|
| | | | | UV-B region | Visible region | Blue region | Transmittance integral in blue region/transmittance integral in visible region |
| | nm | | m²/g | (% · nm) | (% · nm) | (% · nm) | - |
| Example 1 | 31 | 0.820 | 116.4 | 622 | 35913 | 9448 | 0.263 |
| Example 2 | 62 | 0.824 | 74.2 | 601 | 35682 | 9422 | 0.264 |
| Example 3 | 93 | 0.814 | 60.3 | 251 | 35340 | 9113 | 0.258 |
| Example 4 | 37 | 0.870 | 42.9 | 374 | 36220 | 9579 | 0.264 |
| Comparative Example 1 | 131 | 0.837 | 44.0 | 217 | 33006 | 7942 | 0.241 |
| Comparative Example 2 | 212 | 0.826 | 34.9 | 293 | 32618 | 7671 | 0.235 |
| Comparative Example 3 | 311 | 0.697 | 16.8 | 493 | 24013 | 4205 | 0.175 |
| Comparative Example 4 | 38 | 0.470 | 51.3 | 3 | 35191 | 8723 | 0.248 |
| Reference Example 4 | - | - | - | 953 | 32396 | 8243 | 0.254 |

| | | | | | | | |
|---|---|---|---|---|---|---|---|
| Transmittance integral in UV-B region: The smaller the value, the better the ultraviolet protection effect. Transmittance integral in visible region: the larger the value, the higher the transparency. Transmittance integral in blue region: The larger the value, the less bluish the appearance. | | | | | | | |

Table 1 shows production conditions of Examples and Comparative Examples.

Table 2 shows powder properties and transmittance integrals of Examples, Comparative Examples, and Reference Example.

As shown in Table 2, the cosmetic composition with calcium-titanium composite oxide particles having an average particle size of 25 nm or more and 110 nm or less had a transmittance integral of less than 800%•nm in the UV-B region, a transmittance integral of more than 35000%•nm in the visible region, and a transmittance integral of more than 9000%•nm in the blue region, and a value obtained by dividing the transmittance integral in the blue region by the transmittance integral in the visible region was more than 0.250. Comparative Examples 1 to 3 having a particle size of more than 110 nm and Comparative Example 4 using titanium oxide instead of calcium-titanium composite oxide did not satisfy the above conditions.

From the above evaluation results, it is considered that the powder of the present invention has the ability to block UV-B and, when blended into a cosmetic composition, and is highly transparent without taking on a blue tinge.

Sensory tests were conducted on the transparency and blue tinge of the powder of the present invention when blended into a cosmetic composition.

### (Sensory Evaluation of Cosmetic Composition)

Using the powders of Examples and Comparative Examples, W/O sunscreens were prepared by the method described in the section of [Transmittance Integral] above.

### (White Cast)

The obtained sunscreen and sunscreen of Reference Example 1 were applied to the faces of 10 panelists and scored for a white cast according to the following criteria. The higher the score was, the less white cast was left by the sunscreen.

### (Evaluation Criteria)

3 points: No sense of incongruity compared to non-applied area, even when viewed from a distance of 30 cm.
2 points: Incongruous when viewed from a distance of 30 cm, but not from a position 1 m away.
1 point: Incongruous even from a position 1 m or more away.

### (Blue Tinge)

The obtained sunscreen and sunscreen of Reference Example 1 were applied to the faces of 10 panelists and scored for a blue tinge according to the following criteria. The higher the score was, the less the blue tinge was.

### (Evaluation Criteria)

3 points: No blue tinge even when viewed from a distance of 30 cm.
2 points: Seemed bluish white when viewed from a distance of 30 cm, but not noticeable from a position 1 m away.
1 point: Looked bluish white even when viewed from a position 1 m or more away.

### (Criteria for Overall Evaluation)

A: 6 points in total for the white cast and blue tinge scores
B: 4 points or more and less than 6 points in total for the white cast and blue tinge scores
C: Less than 4 points in total for the white cast and blue tinge scores

**[Table 3]**

| Sensory test item | White cast | Blue tinge | Overall evaluation |
|---|---|---|---|
| Example 1 | 3 | 3 | A |
| Example 2 | 3 | 3 | A |
| Example 3 | 3 | 3 | A |
| Example 4 | 3 | 3 | A |
| Comparative Example 1 | 2 | 1 | C |
| Comparative Example 2 | 2 | 1 | C |
| Comparative Example 3 | 1 | 1 | C |
| Comparative Example 4 | 3 | 2 | B |
| Reference Example 4 | 2 | 2 | B |

From the results of the sensory evaluation, it was found that the sunscreen using the powder composed of calcium-titanium composite oxide particles having an average particle size of 25 nm or more and 110 nm or less was less likely to leave a white cast without taking on a blue tinge. The sunscreen was comparable to the existing sunscreen using titanium dioxide of Comparative Example 4 in the tendency to hardly leave a white cast and show a blue tinge. Furthermore, as compared with Reference Example 1, which is a commercially available product, both the tendency to hardly leave a white cast and to show a blue tinge were improved.

From the above evaluation results, the powder composed of the calcium-titanium composite oxide to be blended into the cosmetic composition of the present invention, as the evaluation results of the transmittance integral, hardly left a white cast without taking on a blue tinge when blended into the cosmetic composition.

A skin cosmetic is the main aspect of the present invention. Formulation Examples are described below.

### [Formulation Example 1]

### [Production of Powder Foundation]

Ingredients 1 to 13 below were uniformly pulverized (step A), and ingredients 15 to 17 were uniformly mixed together and added to the mixed-pulverized product obtained in step A to homogenize the mixture (step B). Furthermore, ingredient 14 was added and press-molded into a mold to obtain a powder foundation (step C).

When applied to the skin, the obtained powder foundation did not leave a white cast or show a blue tinge. When the applied area was checked at a later date, sunburn was minimal.

| (Ingredient): blending ratio | (g/kg) |
|---|---|
| 1. Caprylylsilane-treated mica (Note 1): | 400 |
| 2. Caprylylsilane-treated red iron oxide (Note 1): | 50 |
| 3. Silicone-treated talc (Note 2): | balance |
| 4. Silicone-treated powder described in Example 2 (Note 2): | 50 |
| 5. Silicone-treated fine particulate titanium oxide (Note 2): | 50 |
| 6. Silicone-treated barium sulfate (Note 2): | 100 |
| 7. Silicone-treated iron oxide red (Note 2): | 4 |
| 8. Silicone-treated yellow iron oxide (Note 2): | 20 |
| 9. Silicone-treated amber (Note 2): | 4 |
| 10. Silicone-treated black iron oxide (Note 2): | 1 |
| 11. Phenyl-modified hybrid silicone composite powder (Note 3): | 20 |
| 12. Spherical polymethyl silsesquioxane powder (Note 4): | 5 |
| 13. Antiseptic: | q.s. |
| 14. Perfume: | q.s. |
| 15. Crosslinked dimethylpolysiloxane (Note 5): | 40 |
| 16. Glyceryl trioctanoate: | 20 |
| 17. Squalane: | 10 |

| | |
|---|---|
| (Note 1) Surface treated with AES-3083 manufactured by Shin-Etsu Chemical Co., Ltd. (Note 2) Surface treated with KF-9909 manufactured by Shin-Etsu Chemical Co., Ltd. (Note 3) KSP-300 manufactured by Shin-Etsu Chemical Co., Ltd. (Note 4) KMP-590 manufactured by Shin-Etsu Chemical Co., Ltd. (Note 5) KSG-16 manufactured by Shin-Etsu Chemical Co., Ltd. | |

### [Formulation Example 2]

### (Production of Pressed Powder)

After mixing and pulverizing the following ingredients 1 to 7 (step A), the mixed-pulverized product was transferred to a mixer, ingredients 8 to 12 were added, and the mixture was uniformly stirred and mixed (step B), and pulverized with a sample mill, which was then press-molded on an aluminum plate to obtain a pressed powder (step C).

When applied to the skin, the obtained pressed powder did not leave a white cast or show a blue tinge. When the applied area was checked at a later date, sunburn was minimal.

| (Ingredient): blending ratio | (g/kg) |
|---|---|
| 1. Talc: | balance |
| 2. PMMA (Note 1): | 100 |
| 3. Sericite: | 300 |
| 4. Scaly silica (Note 2): | 30 |
| 5. Powder described in Example 1: | 60 |
| 6. Antiseptic: | q.s. |
| 7. Colorant: | q.s. |
| 8. Octyl methoxycinnamate: | 30 |
| 9. Squalane: | 20 |
| 10. Antiseptic: | q.s. |
| 11. Antioxidant: | q.s. |
| 12. Perfume: | q.s. |

| | |
|---|---|
| (Note 1) Matsumoto Microsphere M-100 manufactured by Matsumoto Yushi-Seiyaku Co., Ltd., 7 µm product (Note 2) SUNLOVELY(R) C manufactured by AGC Si-Tech Co., Ltd. | |

### [Formulation Example 3]

### (Production of Loose Powder)

After mixing and pulverizing the following ingredients 1 to 7 (step A), the mixed-pulverized product was transferred to a mixer, ingredients 8 to 10 were added, and the mixture was uniformly stirred and mixed (step B). Furthermore, the mixture obtained in step B was pulverized with a sample mill and filled to obtain a loose powder (step C).

When applied to the skin, the obtained loose powder did not leave a white cast or show a blue tinge. When the applied area was checked at a later date, sunburn was minimal.

| (Ingredient): blending ratio | (g/kg) |
|---|---|
| 1. Talc: | balance |
| 2. Powder described in Example 4: | 50 |
| 3. Amihope LL: | 30 |
| 4. PMMA (Note 1): | 80 |
| 5. Antiseptic: | q.s. |
| 6. Colorant: | q.s. |
| 7. Squalane: | 10 |
| 8. Antiseptic: | q.s. |
| 9. Antioxidant: | q.s. |
| 10. Perfume: | q.s. |

| | |
|---|---|
| (Note 1) Matsumoto Microsphere S-100 manufactured by Matsumoto Yushi-Seiyaku Co., Ltd., 10 µm product | |

### [Formulation Example 4]

### (Production of Oily Foundation)

After mixing the following ingredients 1 to 6 with a mixer and pulverizing the mixture uniformly (step A), ingredients 7 to 16 were heated to 85°C to dissolve the mixture, and the mixed-pulverized product obtained in step A was added and uniformly stirred (step B). After defoaming, the solids were poured into a tray and slowly cooled to room temperature to obtain an oily foundation (step C).

When applied to the skin, the obtained oily foundation did not leave a white cast or show a blue tinge. When the applied area was checked at a later date, sunburn was minimal.

| (Ingredient): blending ratio | (g/kg) |
|---|---|
| 1. Silicone-treated talc (Note 1): | 53 |
| 2. Silicone-treated powder described in Example 3 (Note 1): | 150 |
| 3. Silicone-treated sericite (Note 1): | 282 |
| 4. Silicone-treated iron oxide red (Note 1): | 5 |
| 5. Silicone-treated yellow iron oxide (Note 1): | 18 |
| 6. Silicone-treated black iron oxide (Note 1): | 2 |
| 7. Candelilla wax: | 10 |
| 8. Carnauba wax: | 10 |
| 9. Ceresin: | 15 |
| 10. Decamethylcyclopentasiloxane: | 140 |
| 11. Isononyl isononanoate: | balance |
| 12. Polyglyceryl diisostearate: | 20 |
| 13. Dextrin palmitate: | 10 |
| 14. Octyl methoxycinnamate: | 30 |
| 15. Antiseptic: | q.s. |
| 16. Antioxidant: | q.s. |

| | |
|---|---|
| (Note 1) Surface treated with KF-96A-50cs manufactured by Shin-Etsu Chemical Co., Ltd. | |

### [Formulation Example 5]

### (Production of Stick Foundation)

The following ingredients 12 to 16 were mixed in a mixer (step A). Ingredients 1 to 11 were weighed into a container capable of holding the entire amount, and heated to 85°C to dissolve the mixture (step B). In addition, ingredients 17 to 21 were weighed into a separate container and dissolved (step C). Then, the mixture obtained in step A was added to the thermally-dissolved mixture obtained in step B and dispersed therein using a stirrer until the resulting mixture became visually uniform, and the thermally-dissolved mixture obtained in step C was further added thereto to emulsify the mixture (step D). After defoaming, the solids were then poured into a mold and slowly cooled to room temperature to obtain a stick foundation (step E).

When applied to the skin, the obtained stick foundation did not leave a white cast or show a blue tinge. When the applied area was checked at a later date, sunburn was minimal.

| (Ingredient): blending ratio | (g/kg) |
|---|---|
| 1. Dimethylpolysiloxane: | 180 |
| 2. Decamethylcyclopentasiloxane: | 300 |
| 3. Octyl methoxycinnamate: | 50 |
| 4. Diisostearyl malate: | 40 |
| 5. Candelilla wax: | 60 |
| 6. Hydrogenated jojoba ester: | 40 |
| 7. Cetyl dimethicone copolyol: | 20 |
| 8. Sorbitan sesquiisostearate: | 5 |
| 9. Oxidation inhibitor: | q.s. |
| 10. Antiseptic: | q.s. |
| 11. Perfume: | q.s. |
| 12. Silicone-treated coloring agent (Note 1): | 5 |
| 13. Silicone-treated powder described in Example 1 (Note 1): | 85 |
| 14. Silicone-treated talc (Note 1): | 60 |
| 15. Silicone-treated mica (Note 1): | 20 |
| 16. Methyl polymethacrylate: | 20 |
| 17. Purified water: | balance |
| 18. Sodium citrate: | 3 |
| 19. 1,3-Butylene glycol: | 30 |
| 20. Glycerin: | 20 |
| 21. Antiseptic: | q.s. |

| | |
|---|---|
| (Note 1) Surface treated with KF-99P manufactured by Shin-Etsu Chemical Co., Ltd. | |

### [Formulation Example 6]

### (Production of W/O Emulsified-type Foundation)

After stirring and mixing the following ingredients 12 to 14 using a mixer (step A), ingredients 1 to 11 were added and dispersed therein using a stirrer until the resulting mixture became visually uniform (step B). Meanwhile, ingredients 15 to 19 were heated and dissolved in a separate container (step C). Then, the thermally-dissolved mixture obtained in step C was added to the dispersion product obtained in step B to emulsify the mixture and then cooled to room temperature to obtain a W/O emulsified-type foundation (step D).

When applied to the skin, the obtained W/O emulsified-type foundation did not leave a white cast or show a blue tinge. When the applied area was checked at a later date, sunburn was minimal.

| (Ingredient): blending ratio | (g/kg) |
|---|---|
| 1. POE-modified silicone (Note 1): | 8 |
| 2. Polyglyceryl polyricinoleate: | 5 |
| 3. Neopentyl glycol dicaprate: | 30 |
| 4. Squalane: | 10 |
| 5. Pentaerythrityl tetraoctanoate: | 20 |
| 6. Stearoyl inulin (Note 2): | 10 |
| 7. Octyl methoxycinnamate: | 40 |
| 8. Decamethylcyclopentasiloxane: | 154 |
| 9. Antiseptic: | q.s. |
| 10. Antioxidant: | q.s. |
| 11. Perfume: | q.s. |
| 12. Silicone-treated powder described in Example 4 (Note 3): | 80 |
| 13. Silicone-treated talc (Note 3): | 57 |
| 14. Silicone-treated coloring agent (Note 3): | 10 |
| 15. Purified water: | balance |
| 16. 1,3-Butylene glycol: | 60 |
| 17. Glycerin: | 10 |
| 18. Sodium chloride: | 10 |
| 19. Antiseptic: | q.s. |

| | |
|---|---|
| (Note 1) Product with an HLB value of 4.5 (Note 2) Rheopearl (R) ISK manufactured by Chiba Flour Milling Co., Ltd. (Note 3) Surface treated with KF-9901 manufactured by Shin-Etsu Chemical Co., Ltd. | |

### [Formulation Example 7]

### (Production of O/W Emulsified-type Foundation)

The following ingredients 1 to 7 were heated at 85°C to dissolve the mixture (step A). Separately, ingredients 8 to 10 were mixed and pulverized (step B). Furthermore, Ingredients 11 to 15 were heated to 85°C, dissolved, and mixed (step C). Then, the mixed-pulverized product obtained in step B was added to the thermally-dissolved mixture obtained in step A, and dispersed therein using a stirrer until the resulting mixture became visually uniform. The dissolved mixture obtained in step C was gradually added thereto to emulsify the mixture and cooled to room temperature while stirring. Next, the cooled mixture was filled into an appropriate container to obtain an O/W emulsified-type foundation (step D).

When applied to the skin, the obtained O/W emulsified-type foundation did not leave a white cast or show a blue tinge. When the applied area was checked at a later date, sunburn was minimal.

| (Ingredient): blending ratio | (g/kg) |
|---|---|
| 1. Stearic acid: | 4 |
| 2. Isostearic acid: | 3 |
| 3. Cetyl 2-ethylhexanoate: | 40 |
| 4. Liquid paraffin: | 110 |
| 5. Polyoxyethylene(10) stearyl ether: | 20 |
| 6. Cetyl alcohol: | 3 |
| 7. Antiseptic: | 2 |
| 8. Talc: | 150 |
| 9. Coloring agent: | 40 |
| 10. Powder described in Example 2: | 50 |
| 11. Triethanolamine: | 4 |
| 12. Propylene glycol: | 50 |
| 13. Purified water: | 521 |
| 14. Antiseptic: | 2 |
| 15. Antioxidant: | 1 |

### [Formulation Example 8]

### (Production of W/O Liquid Foundation)

The following ingredients 8 to 12 were uniformly mixed (step A), and a part of ingredient 4 and ingredient 13 were mixed together, added to the mixture obtained in step A, and dispersed therein using a stirrer until the resulting mixture became visually uniform (step B). Separately, ingredients 1 to 3, the rest of ingredient 4, and ingredients 5 to 7 were mixed and dispersed using a stirrer until the resulting mixture became visually uniform (step C). Furthermore, ingredients 14 to 18, and ingredient 20 were mixed and dispersed using a stirrer until the resulting mixture became visually uniform (step D). The mixture obtained in step D was gradually added with stirring to the dispersion product obtained in step C to emulsify the mixture, and the dispersion product obtained in step B and ingredient 19 were further added thereto to obtain a W/O liquid foundation (step E).

When applied to the skin, the obtained W/O liquid foundation did not leave a white cast or show a blue tinge. When the applied area was checked at a later date, sunburn was minimal.

| (Ingredient): blending ratio | (g/kg) |
|---|---|
| 1. Crosslinked polyether-modified silicone (Note 1): | 30 |
| 2. Crosslinked dimethylpolysiloxane (Note 2): | 50 |
| 3. Branched polyether-modified silicone (Note 3): | 20 |
| 4. Decamethylcyclopentasiloxane: | 211 |
| 5. Cetyl isooctanoate: | 50 |
| 6. Dimethylpolysiloxane (Note 4): | 65 |
| 7. Dimethyldistearyl ammonium hectorite: | 12 |
| 8. Silicone-treated powder described in Example 3 (Note 5): | 50 |
| 9. Silicone-treated pigment-grade titanium oxide (Note 5): | 50 |
| 10. Silicone-treated red iron oxide (Note 5): | 4 |
| 11. Silicone-treated yellow iron oxide (Note 5): | 10 |
| 12. Silicone-treated black iron oxide (Note 5): | 1 |
| 13. Acrylic silicone resin dissolved product (Note 6): | 20 |
| 14. 1,3-Butylene glycol: | 50 |
| 15. Xanthan gum (Note 7): | 50 |
| 16. Sodium citrate: | 2 |
| 17. Sodium chloride: | 5 |
| 18. Antiseptic: | q.s. |
| 19. Perfume: | q.s. |
| 20. Purified water: | balance |

| | |
|---|---|
| (Note 1) KSG-210 manufactured by Shin-Etsu Chemical Co., Ltd. (Note 2) KSG-15 manufactured by Shin-Etsu Chemical Co., Ltd. (Note 3) KF-6028P manufactured by Shin-Etsu Chemical Co., Ltd. (Note 4) Product at 6 mm²/sec (25°C) (Note 5) Surface treated with KF-9909 manufactured by Shin-Etsu Chemical Co., Ltd. (Note 6) KP-575 manufactured by Shin-Etsu Chemical Co., Ltd. (Note 7) 20 g/kg of aqueous solution | |

### [Formulation Example 9]

### (Production of 2WAY Cake Foundation)

Ingredients 1 to 7 were mixed with a Henschel mixer (step A), and thermally-dissolved ingredients 8 to 11 were mixed therein (step B), and the mixture was pulverized with an atomizer and press-molded into an aluminum dish to obtain a 2-way foundation of interest (step C).

When applied to the skin, the obtained 2-way foundation did not leave a white cast or show a blue tinge. When the applied area was checked at a later date, sunburn was minimal.

| (Ingredient): blending ratio | (g/kg) |
|---|---|
| 1. Dimethicone-treated talc: | balance |
| 2. Dimethicone-treated powder described in Example 1: | 100 |
| 3. Dimethicone-treated mica: | 200 |
| 4. Dimethicone-treated sericite: | 360 |
| 5. Nylon powder: | 100 |
| 6. Dimethicone-treated red iron oxide: | 29 |
| 7. Dimethicone 1000cs: | 60 |
| 8. Isotridecyl isononanoate: | 30 |
| 9. Squalane: | 30 |
| 10. Tocopherol: | 1 |
| 11. 1,3-Butylene glycol: | 10 |

### [Formulation Example 10]

### (Production of Oily Cake Foundation)

Ingredients 1 to 5 were mixed with a Henschel mixer and uniformly pulverized (step A). Ingredients 6 to 12 were heated and dissolved, the powder portion was added, and the mixture was uniformly stirred (step B). After defoaming, the bulk was poured into a tray and slowly cooled to room temperature to obtain an oily cake foundation of interest (step C).

When applied to the skin, the obtained oily cake did not leave a white cast or show a blue tinge. When the applied area was checked at a later date, sunburn was minimal.

| (Ingredient): blending ratio | (g/kg) |
|---|---|
| 1. Dimethicone-treated talc: | balance |
| 2. Dimethicone-treated pigment described in Example 4: | 130 |
| 3. Dimethicone-treated sericite: | 280 |
| 4. Dimethicone-treated red iron oxide: | 27 |
| 5. Dimethicone-treated black iron oxide: | 1 |
| 6. Candelilla wax: | 10 |
| 7. Carnauba wax: | 10 |
| 8. Ceresin: | 15 |
| 9. Cyclopentasiloxane: | 140 |
| 10. Isononyl isononanoate: | 250 |
| 11. Polyglyceryl diisostearate: | 20 |
| 12. Ethylhexyl methoxycinnamate: | 30 |

### [Formulation Example 11]

### (Production of Suncut Cream)

The following ingredient 7 was added to a part of ingredient 5 to obtain a uniform mixture, and ingredients 8 and 9 were added and dispersed therein using a bead mill (step A). Separately, ingredients 1 to 4, the rest of ingredient 5, and ingredient 6 were uniformly mixed (step B). Furthermore, ingredients 10 to 12, and ingredient 14 were dispersed using a stirrer until the resulting mixture became visually uniform (step C). Subsequently, the dispersion product obtained in step C was added to the mixture obtained in step B to emulsify the mixture, and the dispersion product obtained in step A and ingredient 13 were added thereto to obtain a suncut cream (step D).

When applied to the skin, the obtained sun cut cream did not leave a white cast or show a blue tinge. When the applied area was checked at a later date, sunburn was minimal.

| (Ingredient): blending ratio | (g/kg) |
|---|---|
| 1. Crosslinked polyether-modified silicone (Note 1): | 30 |
| 2. Crosslinked dimethylpolysiloxane (Note 2): | 20 |
| 3. Alkyl-modified branched polyether-modified silicone (Note 3): | 10 |
| 4. Neopentyl glycol dioctanate: | 50 |
| 5. Decamethylcyclopentasiloxane: | 175 |
| 6. Octyl methoxycinnamate: | 60 |
| 7. Acrylic silicone resin dissolved product (Note 4): | 100 |
| 8. Caprylylsilane-treated fine particulate zinc oxide (Note 5): | 200 |
| 9. Caprylylsilane-treated powder described in Example 1 (Note 5): | 30 |
| 10. 1,3-Butylene glycol: | 20 |
| 11. Sodium citrate: | 2 |
| 12. Sodium chloride: | 5 |
| 13. Perfume: | q.s. |
| 14. Purified water: | balance |

| | |
|---|---|
| (Note 1) KSG-240 manufactured by Shin-Etsu Chemical Co., Ltd. (Note 2) KSG-15 manufactured by Shin-Etsu Chemical Co., Ltd. (Note 3) KF-6038 manufactured by Shin-Etsu Chemical Co., Ltd. (Note 4) KP-575 manufactured by Shin-Etsu Chemical Co., Ltd. (Note 5) Surface treated with AES-3083 manufactured by Shin-Etsu Chemical Co., Ltd. | |

### [Formulation Example 12]

### (Preparation of W/O Emulsion-type Sunscreen)

After ingredients 1 to 5 were uniformly mixed, ingredient 6 was uniformly dispersed (step A). Then, ingredients 7 to 11 were uniformly mixed (step B). The mixture obtained in step B was added to the dispersion product obtained in step A to emulsify the mixture to obtain a W/O emulsion-type sunscreen (step C).

When applied to the skin, the obtained W/O emulsion-type sunscreen did not leave a white cast or show a blue tinge. When the applied area was checked at a later date, sunburn was minimal.

| (Ingredient): blending ratio | (g/kg) |
|---|---|
| 1. Crosslinked polyether-modified silicone (Note 1): | 20 |
| 2. Crosslinked dimethylpolysiloxane (Note 2): | 30 |
| 3. Decamethylcyclopentasiloxane: | 135 |
| 4. Dimethylpolysiloxane (6 mm²/sec (25°C)): | 70 |
| 5. Silicone-treated powder described in Example 3 (Note 3): | 250 |
| 6. Silicone-treated talc: | 40 |
| 7. 1,3-Butylene glycol: | 50 |
| 8. Sodium citrate: | 4 |
| 9. Sodium chloride: | 5 |
| 10. Antiseptic: | q.s. |
| 11. Purified water: | balance |

| | |
|---|---|
| (Note 1) KSG-210 manufactured by Shin-Etsu Chemical Co., Ltd. (Note 2) KSG-15 manufactured by Shin-Etsu Chemical Co., Ltd. (Note 3) Surface treated with KF-9909 manufactured by Shin-Etsu Chemical Co., Ltd. | |

### [Formulation Example 13]

### (Production of O/W Emulsion-type Sunscreen)

The following ingredients 1 to 9 were uniformly mixed (step A). Next, ingredients 10 to 15 were uniformly mixed (step B). The mixture obtained in step A was added to the mixture obtained in step B to emulsify the mixture with stirring to obtain an O/W emulsion-type sunscreen (step C).

When applied to the skin, the obtained O/W emulsion-type sunscreen did not leave a white cast or show a blue tinge. When the applied area was checked at a later date, sunburn was minimal.

| (Ingredient): blending ratio | (g/kg) |
|---|---|
| 1. Lauryl betaine: | 1 |
| 2. Isostearic acid: | 2 |
| 3. Diethylhexyl succinate: | 145 |
| 4. Cyclomethicone: | 20 |
| 5. Dimethicone: | 20 |
| 6. 2-Ethylhexyl 2-ethylhexanoate: | 20 |
| 7. Behenyl alcohol: | 1 |
| 8. Batyl alcohol: | 1 |
| 9. Polyoxybutylene polyoxypropylene glycol: | 20 |
| 10. Ethanol: | 80 |
| 11. Triethanolamine: | 3 |
| 12. EDTA-3Na: | 1 |
| 13. Phenoxyethanol: | 3 |
| 14. Powder described in Example 1: | 80 |
| 15. Purified water: | balance |

### [Formulation Example 14]

### (Preparation of W/O cream sunscreen)

Ingredients 1 to 7 below were uniformly mixed (step A). Then, ingredients 8 to 13 and 15 were uniformly mixed (step B). The mixture obtained in step B was added to the mixture obtained in step A to emulsify the mixture with stirring, followed by addition of ingredient 14, to obtain a W/O cream sunscreen (step C).

When applied to the skin, the obtained W/O cream sunscreen did not leave a white cast or show a blue tinge. It was confirmed that the W/O cream sunscreen was smooth without squeakiness, spread lightly, had excellent adhesion, was well-fitted, and was very excellent in usability and stability. In addition, when the applied area was checked at a later date, sunburn was minimal.

| (Ingredient): blending ratio | (g/kg) |
|---|---|
| 1. Crosslinked alkyl/polyether-modified silicone (Note 1): | 30 |
| 2. Crosslinked alkyl-modified dimethylpolysiloxane (Note 2): | 40 |
| 3. Alkyl-modified branched polyether-modified silicone (Note 3): | 10 |
| 4. Meadowfoam oil: | 35 |
| 5. Macadamia nut oil: | 50 |
| 6. Jojoba oil: | 100 |
| 7. Hybrid silicone composite powder (Note 4): | 30 |
| 8. Powder described in Example 4: | 50 |
| 9. 1,3-Butylene glycol: | 80 |
| 10. Glycine: | 30 |
| 11. Sodium citrate: | 2 |
| 12. Sodium chloride: | 5 |
| 13. Antiseptic: | q.s. |
| 14. Perfume: | q.s. |
| 15. Purified water: | balance |

| | |
|---|---|
| (Note 1) KSG-340 manufactured by Shin-Etsu Chemical Co., Ltd. (Note 2) KSG-44 manufactured by Shin-Etsu Chemical Co., Ltd. (Note 3) KF-6038 manufactured by Shin-Etsu Chemical Co., Ltd. (Note 4) KSP-100 manufactured by Shin-Etsu Chemical Co., Ltd. | |

### [Formulation Example 15]

### (Production of O/W Cream Sunscreen)

Ingredients 1 to 7 were heated and mixed to homogenize (step A) the mixture. Then, ingredients 8 to 13 and 15 were uniformly mixed (step B). The mixture obtained in step A was added to the mixture obtained in step B to emulsify the mixture with stirring, followed by addition of ingredient 14, to obtain an O/W cream sunscreen (step C).

When applied to the skin, the obtained O/W cream sunscreen did not leave a white cast or show a blue tinge. It was confirmed that the O/W cream sunscreen was smooth without stickiness and oiliness, spread lightly, had excellent adhesion, was well-fitted, maintained makeup well, showed little change with temperature and time, and was very excellent in usability and stability. In addition, when the applied area was checked at a later date, sunburn was minimal.

| (Ingredient): blending ratio | (g/kg) |
|---|---|
| 1. Crosslinked dimethylpolysiloxane (Note 1): | 50 |
| 2. Glyceryl triisostearate: | 80 |
| 3. Cetanol: | 5 |
| 4. Stearic acid: | 10 |
| 5. Glyceryl monostearate: | 5 |
| 6. Sorbitan sesquioleate: | 5 |
| 7. Coupling agent-treated powder described in Example 4 (Note 2): | 50 |
| 8. Polyoxyethylene sorbitan monooleate: | 10 |
| 9. Triethanolamine: | 5 |
| 10. Carbomer (10 g/kg of aqueous solution): | 200 |
| 11. Locust bean gum (20 g/kg of aqueous solution): | 50 |
| 12. 1,3-Butylene glycol: | 70 |
| 13. Antiseptic: | q.s. |
| 14. Perfume: | q.s. |
| 15. Purified water: | balance |

| | |
|---|---|
| (Note 1) KSG-15 manufactured by Shin-Etsu Chemical Co., Ltd. (Note 2) Surface treated with OFS-6341 manufactured by Dow Chemical Japan Ltd. | |

### [Formulation Example 16]

### (Production of O/W Gel Sunscreen)

The following ingredients 1 to 4 were uniformly mixed (step A). After ingredients 6 and 7 were mixed uniformly, ingredient 5 was then added and mixed uniformly (step B). The mixture obtained in step A was added to the mixture obtained in step B to emulsify the mixture with stirring to obtain an O/W gel sunscreen (step C).

When applied to the skin, the obtained O/W gel sunscreen did not leave a white cast or show a blue tinge. It was confirmed that the O/W gel sunscreen was smooth without stickiness or oiliness, spread lightly, had excellent adhesion, was well-fitted, and was very excellent in usability and stability. In addition, when the applied area was checked at a later date, sunburn was minimal.

| (Ingredient): blending ratio | (g/kg) |
|---|---|
| 1. Polyhydroxystearic acid: | 3 |
| 2. Ethylhexyl methoxycinnamate: | 47 |
| 3. Liquid paraffin: | 295 |
| 4. Powder described in Example 1: | 50 |
| 5. Sodium acrylate/sodium acryloyldimethyl taurate copolymer/Isohexadecane/polysorbate 80 (Note 1): | 5 |
| 6. 1,3-Butylene glycol: | 60 |
| 7. Purified water: | balance |

| | |
|---|---|
| (Note 1) SIMULGEL EG QD manufactured by Seppic S.A. | |

### [Formulation Example 17]

### (Production of Stick Sunscreen)

Ingredients 1 to 4 were heated from 85°C to 90°C and mixed uniformly (step A). Ingredients 5 and 6 were dissolved in ingredient 7 and homogenized (step B). The following ingredients 8 to 10 below were uniformly mixed (step C). While stirring, the mixture obtained in step B was added to the mixture obtained in step A and mixed uniformly, cooled from 70°C to 80°C, and the mixture obtained in step C is added thereto and mixed uniformly. Ingredients 11 to 15 were then added, and this mixture was filled into an appropriate container and cooled to obtain a stick sunscreen (step D).

When applied to the skin, the obtained stick sunscreen did not leave a white cast or show a blue tinge. It was confirmed that the stick sunscreen had excellent adhesion and was well-fitted without squeakiness. In addition, when the applied area was checked at a later date, sunburn was minimal.

| (Ingredient): blending ratio | (g/kg) |
|---|---|
| 1. Paraffin wax: | 280 |
| 2. Cetyl alcohol: | 5 |
| 3. White wax: | 30 |
| 4. Carnauba wax: | 10 |
| 5. Methylparaben: | 1 |
| 6. Propylparaben: | 1 |
| 7. Isopropyl myristate: | 10 |
| 8. Oxybenzone: | 35 |
| 9. Octinoxate: | 75 |
| 10. Avobenzone: | 30 |
| 11. Perfume: | 10 |
| 12. White petrolatum: | 433 |
| 13. Lanolin: | 10 |
| 14. Isopyl lanolate: | 20 |
| 15. Powder described in Example 2: | 50 |

### [Formulation Example 18]

### (Production of Spray Sunscreen)

Ingredients 1 to 7 were mixed and dissolved at 80°C to homogenize (step A) the mixture. Ingredients 8 to 10 and 13 were mixed and dissolved at 80°C to homogenize the mixture (step B). The mixture obtained in step B was added to the mixture obtained in step A and emulsified. After the mixture was cooled to 30°C, ingredients 11 and 12 were stirred and mixed. A pump-type spray container was filled with the obtained emulsion for spraying to obtain a spray sunscreen (step C).

When applied to the skin, the obtained spray sunscreen did not leave a white cast or show a blue tinge. In addition, the spray sunscreen could be sprayed cleanly and was refreshing with little oiliness. In addition, when the applied area was checked at a later date, sunburn was minimal.

| (Ingredient): blending ratio | (g/kg) |
|---|---|
| 1. Ethylhexyl methoxycinnamate (Note 1): | 65 |
| 2. t-Butyl methoxydibenzoylmethane (Note 2): | 6 |
| 3. Decamethylcyclopentasiloxane (Note 3): | 50 |
| 4. Silicone-treated powder described in Example 1 (Note 4): | 70 |
| 5. Caprylyl methicone (Note 5): | 10 |
| 6. Sorbitan stearate (Note 6): | 10 |
| 7. Polysorbate 60: | 2 |
| 8. PEG-11 methyl ether dimethicone: | 7 |
| 9. Acrylic acid-alkyl methacrylate copolymer (Note 7): | 1 |
| 10. 1,3-Butylene glycol: | 30 |
| 11. Ethanol: | 100 |
| 12. Potassium hydroxide: | q.s. |
| 13. Purified water: | balance |

| | |
|---|---|
| (Note 1) NOMUCOAT(R) TAB manufactured by The Nisshin OilliO Group, Ltd. (Note 2) PARSOL(R) 1789 manufactured by DSM Nutrition Japan K.K. (Note 3) KF-995 manufactured by Shin-Etsu Chemical Co., Ltd. (Note 4) Surface treated with KF-9909 manufactured by Shin-Etsu Chemical Co., Ltd. (Note 5) SS-3408 manufactured by Dow Toray Co., Ltd. (Note 6) EMALEX(R) SPE-100S manufactured by Nihon Emulsion Co., Ltd. (Note 7) PEMULEN (R) TR-1 manufactured by Lubrizol Japan Limited | |

### [Formulation Example 19]

### (Production of Suntan Lotion)

Ingredients 2 to 7 were sequentially added to ingredient 1 with stirring at room temperature and stirred until the mixture became uniform to obtain a suntan lotion.

When applied to the skin, the obtained suntan lotion did not leave a white cast without taking on a blue tinge or cause squeakiness. When the applied area was intentionally exposed to sunlight and checked at a later date, the skin had a uniform tan color.

| (Ingredient): blending ratio | (g/kg) |
|---|---|
| 1. Ethylhexyl methoxycinnamate: | 100 |
| 2. Isopropyl myristate: | 100 |
| 3. Coconut oil: | 50 |
| 4. Natural vitamin E: | 1 |
| 5. Silicone-treated powder described in Example 1 (Note 1): | 70 |
| 6. Perfume: | q.s. |
| 7. Liquid paraffin: | balance |

| | |
|---|---|
| (Note 1) Surface treated with KF-9909 manufactured by Shin-Etsu Chemical Co., Ltd. | |

The powder of the present invention can also be used in products other than skin cosmetic compositions.

### [Formulation Example 20]

### (Production of Body Shampoo)

Ingredients 1 to 3 were heated from 70°C to 80°C and dissolved (step A). Subsequently, ingredient 4 was gradually added to carry out saponification (step B). Once the saponification was completed, the remaining ingredients 5 to 9 were added and homogeneously stirred (step C). After the mixture was cooled to 40°C, ingredients 10 to 12, which had been mixed and pulverized, were added thereto, and the mixture was cooled to room temperature with stirring and mixing to obtain a body shampoo of interest (step D).

The obtained body shampoo lathered well and had a smooth feel.

| (Ingredient): blending ratio | (g/kg) |
|---|---|
| 1. Lauric acid: | 115 |
| 2. Myristic acid: | 77 |
| 3. Palmitic acid: | 48 |
| 4. Potassium hydroxide (purity: 480 g/kg): | 122 |
| 5. Lauryl hydroxy sulfobetaine: | 100 |
| 6. Cocamide monoethanolamide: | 10 |
| 7. Ethylene Glycol distearate: | 20 |
| 8. Purified water: | balance |
| 9. EDTA-4Na: | 1 |
| 10. Powder described in Example 4: | 20 |
| 11. Colorant: | 6 |
| 12. Talc: | 10 |

### [Formulation Example 21]

### (Production of Cleansing Foam)

Ingredients 1 to 3 were heated from 70°C to 80°C and dissolved (step A). Subsequently, ingredient 4 was gradually added to carry out saponification (step B). Once the saponification was completed, the remaining ingredients 5 to 13 were added and homogeneously stirred (step C). After the mixture was cooled to 40°C, ingredients 14 to 16, which had been mixed and pulverized, were added thereto, and the mixture was cooled to room temperature with stirring and mixing to obtain a cleansing foam of interest (step D).

The obtained cleansing foam lathered well and had a smooth feel.

| (Ingredient): blending ratio | (g/kg) |
|---|---|
| 1. Stearic acid: | 170 |
| 2. Myristic acid: | 70 |
| 3. Lauric acid: | 30 |
| 4. Potassium hydroxide (purity: 480 g/kg): | 120 |
| 5. PEG-60 glyceryl diisostearate: | 30 |
| 6. Glyceryl stearate: | 20 |
| 7. Sorbitol: | 80 |
| 8. PEG1500: | 100 |
| 9. Cocamidopropyl betaine: | 50 |
| 10. Glycerin: | 180 |
| 11. EDTA-4Na: | 1 |
| 12. Purified water: | balance |
| 13. Pentylene glycol: | 15 |
| 14. Powder described in Example 1: | 20 |
| 15. Colorant: | 3 |
| 16. Talc: | 10 |

### [Formulation Example 22]

### [Production of Cleansing Powder]

Ingredients 1 to 13 were homogeneously mixed with a Henschel mixer to obtain a cleansing powder of interest.

The obtained cleansing powder lathered well and had a smooth feel.

| (Ingredient): blending ratio | (g/kg) |
|---|---|
| 1. Red iron oxide: | 3 |
| 2. Powder described in Example 4: | 20 |
| 3. Talc: | 200 |
| 4. Lauric acid: | 50 |
| 5. Mannitol: | balance |
| 6. Potassium myristate: | 300 |
| 7. Glucose: | 100 |
| 8. Potassium myristoyl glutamate: | 20 |
| 9. Sodium methyl cocoyl taurate: | 30 |
| 10. Betaine: | 10 |
| 11. Tanakura clay (R): | 50 |
| 12. Guar hydroxypropyltrimonium chloride: | 5 |
| 13. Pentylene glycol: | 10 |

### [Formulation Example 23]

### (Production of Film Composition)

A film composition containing the calcium-titanium composite oxide of the present invention was prepared. A part of ingredient 3 was added to ingredient 1 and dispersed therein using a bead mill (step A). Ingredient 2 and the rest of ingredient 3 were mixed, heated, and stirred at 95°C for 10 minutes (step B). The dispersion obtained in step A was added, and after stirring for 5 minutes, heating was terminated. The mixture was poured into a mold (step C) and dried to obtain a film composition (step D).

The obtained film composition was transparent without white or blue tinges. It is considered that a transparent film-like structure having an ability to block UV-B can be obtained by industrially applying this formulation example.

| (Ingredient): blending ratio | (g/kg) |
|---|---|
| 1. Powder described in Example 1: | 100 |
| 2. Polyvinyl alcohol (Note 1): | 100 |
| 3. Purified water: | 800 |

| | |
|---|---|
| (Note 1) Polyvinyl alcohol 500 manufactured by KISHIDA CHEMICAL Co., Ltd. | |

### [Formulation Example 24]

### (Production of Paint)

A paint containing the powder of the present invention was prepared. Ingredients 1 to 4 were mixed with a mixer for 30 to 120 minutes (step A) and then dispersed using a bead mill to obtain a paint (step B).

The obtained paint, when applied onto a transparent glass substrate and held up to light, was transparent without white or blue tinges. It is considered that a paint having an ability to block UV-B can be obtained by industrially applying this formulation example.

| (Ingredient): blending ratio | (g/kg) |
|---|---|
| 1. Acrylic resin: | 300 |
| 2. Powder described in Example 4 after surface treatment with methylhydrogenpolysiloxane: | 300 |
| 3. Toluene: | 200 |
| 4. Isophorone: | 200 |

## Claims

1. A powder to be blended into a cosmetic composition, the powder comprising calcium-titanium composite oxide particles having an average particle size of 25 nm or more and 110 nm or less.

2. The powder according to claim 1, wherein circularity of the particles is 0.80 or more.

3. The powder according to claim 1 or 2, comprising a coating layer of an inorganic and/or organic substance on at least a part of a surface of the particles.

4. A dispersion comprising the powder according to any one of claims 1 to 3 and a disperse medium.

5. A cosmetic composition comprising the powder according to any one of claims 1 to 3 or the dispersion according to claim 4.

6. A cosmetic composition comprising the powder according to any one of claims 1 to 3 or the dispersion according to claim 4 in an amount such that a content of calcium-titanium composite oxide is 300 g/kg or less.
